# FASCICULE DE BREVET EUROPEEN

(11) **EP 4 230 168 B1**
(45) Date de publication et mention de la délivrance du brevet: **06.05.2026**
(21) Numéro de dépôt: 23157802.2
(22) Date de dépôt: 21.02.2023
(51) Int. Cl.: A61B 34/10, A61B 34/20, A61B 90/00, A61B 90/96, A61B 34/30

(54) **SYSTÈME DE CHIRURGIE RACHIDIENNE ROBOTISÉE**
ROBOTISCHES WIRBELSÄULENCHIRURGIESYSTEM
ROBOTIC SPINAL SURGERY SYSTEM

(30) Priorité: 22.02.2022 FR 2201545
(43) Date de publication de la demande: 23.08.2023
(73) Titulaire: S.M.A.I.O, 69800 Saint-Priest (FR)
(72) Inventeur: ROUSSOULY, Jean-Charles, 69250 POLEYMIEUX AU MONT D'OR (FR)
(74) Mandataire: Lavoix

(56) Documents cités:
- WO-A1-2020/055707
- WO-A1-2021/069449
- US-A1- 2018 207 794
- US-A1- 2018 318 035
- US-A1- 2020 222 122

## Description

La présente invention concerne un système de chirurgie rachidienne.

En chirurgie vertébrale, la robotique joue un rôle croissant principalement pour aider le chirurgien à positionner des vis dans les pédicules des vertèbres du patient ou des cages intersomatiques dans les disques intervertébraux du patient. Les technologies couramment utilisées font intervenir un bras robotisé comportant, en tant qu'organe terminal effecteur, un guide de visée qui est typiquement tubulaire. En peropératoire, c'est-à-dire pendant la chirurgie, la colonne vertébrale du patient est scannée et un marqueur optique tridimensionnel est positionné fixement sur la colonne vertébrale. Ce marqueur permet d'établir un repère spatial dans lequel les vis et autres implants sont, grâce à des outils de traitement logiciels développés à cet effet, positionnés virtuellement au sein d'images calibrées de la colonne vertébrale, obtenues par imagerie par rayons X au bloc opératoire en utilisant, par exemple, un scanner intraopératoire ou un amplificateur de brillance. Les coordonnées de positionnement des implants étant fournies dans le repère du marqueur solidaire de la colonne vertébrale, la visualisation de ce marqueur durant la chirurgie par une caméra dont la position est connue par rapport à celle de la station fixe sur laquelle repose le bras robotisé permet de commander ce dernier pour positionner son organe terminal effecteur dans l'espace de telle sorte que le guide de visée que forme cet organe terminal effecteur permet au chirurgien d'effectuer les actes chirurgicaux d'implantation de manière précise et sécurisée. US 2020/0222122 A1 divulgue un système pour former un réseau dynamique traçable, comprenant un élément de base fixé de manière amovible par rapport à un espace de navigation; un élément mobile pouvant se déplacer par rapport à au moins une première position et une deuxième position; un système de suivi de l'élément mobile pour suivre la position de l'élément mobile; un système de suivi comprenant un processeur de suivi et un localisateur, séparés de l'élément de base et de l'élément mobile; et un élément traçable configuré pour être suivi par le système de suivi et dans lequel l'élément traçable est mobile par l'élément mobile; dans lequel le processeur de suivi est configuré pour déterminer la position de l'élément traçable dans au moins la première position et la deuxième position. Un autre exemple de ce genre de système est divulgué dans US 2020/222121 A1 où sont associés un robot d'assistance chirurgicale et un dispositif de navigation. Le dispositif de navigation permet de suivre dans l'espace des marqueurs optiques tridimensionnels, notamment un marqueur porté par le robot, un marqueur porté par un organe terminal effecteur du robot, un marqueur fixé au patient, un marqueur porté par un instrument à main, et un marqueur porté par un dispositif d'imagerie, notamment un scanner intraopératoire. A cet effet, le dispositif de navigation inclut un localisateur optique multi-caméras qui fournit des images où apparaissent les différents marqueurs précités. Le marqueur fixé au patient inclut un organe de fixation, tel qu'une pince, à même de s'accrocher à l'une ou l'autre des apophyses épineuses de la colonne vertébrale du patient, de sorte que la colonne du patient peut être suivie par le dispositif de navigation dans un repère spatial défini par ce marqueur. Afin de positionner le repère spatial défini par le marqueur fixé au patient dans un repère de navigation, il est nécessaire que l'utilisateur identifie dans le repère de navigation l'emplacement de ce marqueur, et ce à l'aide de l'outil à main. Puis des moyens de traitement du système de navigation calculent une carte pour corréler entre eux les différents repères spatiaux. Des considérations similaires s'appliquent aux différents autres marqueurs, notamment le marqueur porté par l'organe terminal effecteur du robot. Dès lors, on comprend que le système de US 2020/222121 A1 est capable de déterminer, de manière peropératoire, le positionnement relatif de l'organe terminal effecteur, du marqueur fixé au patient et des images de la colonne du patient, prises par le dispositif d'imagerie intraopératoire.

Néanmoins, cette technique souffre de plusieurs limites. Premièrement, elle requiert l'utilisation de dispositifs d'imagerie intraopératoires par rayons X, induisant par là-même de lourds investissements pour l'établissement de soins, d'irradiation systématique du patient et de l'équipe chirurgicale, et des contraintes de fonctionnement, liées au port de tabliers en plomb, aux déplacements dans le bloc opératoire pour éviter l'irradiation, etc. Deuxièmement, elle oblige le chirurgien, selon un processus chronophage, à reconnaître chaque instrument par la caméra, ce qui pose des problèmes d'occlusion quand un objet fait écran entre la caméra et l'objet que la caméra suit et dont elle est censée indiquer en permanence la position par rapport au marqueur fixé à la colonne vertébrale du patient. Si le patient vient à bouger même légèrement, le chirurgien est obligé de recommencer tout le processus de reconnaissance des instruments, voire de réaliser un nouveau scanner complet du patient.

Dès lors, des techniques de repositionnement, basées sur des caméras de très haute précision, souvent à infrarouge, ont été récemment développées. Elles permettent de photographier en peropératoire de manière très précise la surface de la zone anatomique concernée pour en comparer localement la texture avec celle du même objet scanné en préopératoire. Les images du scanner préopératoire sont alors positionnées dans l'espace en superposition de la zone photographiée comme si elles avaient été scannées au bloc opératoire. Il est alors possible de commander un bras robotisé selon des trajectoires planifiées qui sont recalées par rapport aux vertèbres. Si elles diminuent l'irradiation durant la chirurgie et permettent une réacquisition instantanée du positionnement relatif en cas de mouvement inopiné du marqueur fixé à la colonne vertébrale du patient, ces techniques de repositionnement nécessitent le développement d'algorithmes d'analyses d'images complexes et le recours à des caméras très onéreuses, sans changer fondamentalement la donne concernant la lourdeur de la phase initiale au bloc opératoire.

De plus, ces différentes techniques présentent des problèmes de précision, dans le sens où plus la zone d'implantation est éloignée du marqueur fixé à la colonne vertébrale du patient, mois la précision de positionnement est importante en raison des mouvements des vertèbres entre elles, qui peuvent différer des mouvements suivis par le marqueur fixé à un segment vertébral distinct. En raison de ce manque de précision, les actions effectuées par le robot sont souvent limitées au positionnement d'un guide de visée, charge au chirurgien d'effectuer l'action chirurgicale proprement dite, telle qu'une action de perçage, sans gain de temps par rapport à un geste réalisé sans robot, et parfois avec des risques de glissement de l'outil chirurgical sur la vertèbre.

Le but de la présente invention est de proposer une nouvelle approche de la chirurgie rachidienne robotisée qui, tout en étant simple et peu onéreuse à mettre en œuvre, permet de positionner le bras robotisé de façon précise et sécurisée par rapport aux vertèbres d'un patient à opérer.

A cet effet, l'invention a pour objet un système de chirurgie rachidienne, telle que définie à la revendication 1.

Est également présenté ici un procédé de chirurgie rachidienne, qui est avantageusement mis en œuvre par le système de chirurgie rachidienne conforme à l'invention, et dans lequel :
- on dispose de données cartographiques préopératoires relatives à une ou des vertèbres d'un patient à opérer,
- on fabrique un élément vertébral de localisation qui est pourvu :
   - d'une face d'accouplement qui est spécifique au patient, en étant congruente avec une partie osseuse prédéterminée de la ou une des vertèbres, de manière à pouvoir accoupler l'élément vertébral de localisation à la vertèbre dans une position fixe unique, en appliquant la face d'accouplement en contact sur mesure avec la matière osseuse de cette vertèbre de manière que la face d'accouplement recouvre la partie osseuse prédéterminée de la vertèbre, en épousant des reliefs osseux de la partie osseuse prédéterminée et en coopérant par contact avec ces reliefs osseux pour placer l'élément vertébral de localisation sur la vertèbre dans ladite position fixe unique, et
   - d'un marqueur optique tridimensionnel, définissant un repère spatial tridimensionnel qui est lié fixement à la face d'accouplement de sorte que, lorsque l'élément vertébral de localisation est accouplé à la vertèbre, ledit repère spatial tridimensionnel est lié fixement à la vertèbre selon un positionnement relatif résultant de l'accouplement de l'élément vertébral de localisation à la vertèbre dans ladite position fixe unique,
- on dispose également d'un robot comprenant un bras qui est mobile par rapport à une station fixe du robot, ainsi qu'un organe terminal effecteur qui est porté par le bras et qui est adapté pour être appliqué en peropératoire sur la vertèbre,
- après que la vertèbre ait été mise à nu, l'élément vertébral de localisation est accouplé en peropératoire à la vertèbre dans ladite position fixe unique tandis que le marqueur de l'élément vertébral de localisation est observé par un dispositif de capture optique qui est au moins partiellement porté par l'organe terminal effecteur et qui déduit en temps réel des données de positionnement concernant le positionnement relatif entre ce marqueur et l'organe terminal effecteur, et
- à partir des données cartographiques préopératoires et à partir des données de positionnement, on détermine en temps réel le positionnement relatif entre l'organe terminal effecteur et la vertèbre, en calculant, dans ledit repère spatial tridimensionnel, la position de l'organe terminal effecteur et en comparant cette position de l'organe terminal effecteur avec une région de l'espace occupée par la vertèbre telle que modélisée par lesdites données cartographiques préopératoires.

Ainsi, l'invention s'appuie sur des données cartographiques préopératoires relatives à la ou chaque vertèbre d'un patient à opérer, ces données étant typiquement issues d'images de scanners préopératoires. Comme détaillé par la suite, ces données cartographiques préopératoires sont avantageusement segmentées, pour isoler la vertèbre, et/ou font avantageusement l'objet de sélections relatives à des régions anatomiquement remarquables de la vertèbre et/ou sont avantageusement couplées à des données de planification relatives à un acte chirurgical à réaliser sur la vertèbre. Dans tous les cas, l'invention met à profit au moins une partie de ces données cartographiques préopératoires pour que soit élaboré et fabriqué un élément vertébral de localisation : d'une part, cet élément vertébral de localisation est spécifique au patient, plus précisément à la vertèbre concernée de ce dernier, en ayant une face d'accouplement qui permet de lier fixement l'élément vertébral de localisation à la vertèbre dans une position unique, par coopération en contact sur mesure avec la matière osseuse de la vertèbre, en particulier la matière osseuse de l'une ou plusieurs des régions anatomiquement remarquables précitées ; d'autre part, l'élément vertébral de localisation permet de localiser dans l'espace la vertèbre de manière optique, et ce grâce à un marqueur optique tridimensionnel permettant de définir un repère spatial qui est lié fixement à la vertèbre lorsque, lors de la chirurgie, l'élément vertébral de localisation est accouplé à la vertèbre. L'invention repose également sur l'utilisation, au bloc opératoire, d'à la fois un robot, dont le bras mobile motorisé est muni d'un organe terminal effecteur, et d'un dispositif de capture optique qui est au moins partiellement porté par l'organe terminal effecteur. Ce dispositif de capture optique donne, en quelque sorte, des yeux au robot dans le sens où ce dispositif permet de déterminer en temps réel le positionnement relatif entre l'organe terminal effecteur du robot et le marqueur de l'élément vertébral de localisation accouplé à la vertèbre, moyennant l'observation de ce marqueur par le dispositif de capture optique. En utilisant à la fois les données cartographiques préopératoires et les données relatives au positionnement entre l'organe terminal effecteur et le marqueur de l'élément vertébral de localisation, l'invention prévoit de calculer en temps réel le positionnement relatif entre cet organe terminal effecteur et la vertèbre ; autrement dit, la position de l'organe terminal effecteur est calculée en temps réel dans le repère qui est défini par le marqueur de l'élément vertébral de localisation et est lié fixement à la vertèbre telle que modélisée dans ce même repère par les données cartographiques préopératoires. L'invention permet ainsi de repérer, en temps réel et avec précision, l'organe terminal effecteur par rapport à la vertèbre, plus précisément par rapport à la modélisation préopératoire de la vertèbre, de manière simple et économique, notamment en évitant d'utiliser en peropératoire aussi bien un dispositif d'imageries par rayons X, tel qu'un amplificateur de brillance ou un scanner intraopératoire, que des algorithmes complexes d'analyse d'images à rapprocher. Grâce à l'invention, il est alors possible de faire suivre au robot des instructions de commande qui tiennent compte, en temps réel, de la position de la vertèbre par rapport à l'organe terminal effecteur du robot ; en particulier, le déplacement peropératoire de l'organe terminal effecteur aux fins de réaliser ou d'assister un acte chirurgical peut alors tenir compte de tout mouvement éventuel de la vertèbre, lié par exemple à la respiration du patient, à des spasmes du patient ou à un choc externe inopiné subi par la table d'opération sur laquelle repose le patient. En pratique, comme expliqué plus en détail par la suite, le dispositif de capture optique appartenant au système conforme à l'invention peut prendre diverses formes de réalisation ; une forme de réalisation particulièrement avantageuse, notamment de par sa simplicité et ses performances, consiste à ce que ce dispositif de capture optique comprenne une caméra tridimensionnelle qui est portée par l'organe terminal effecteur du robot et qui, à elle seule, fournit les données relatives au positionnement entre cet organe terminal effecteur et le marqueur de l'élément vertébral de localisation. Dans tous les cas, et comme également expliqué plus en détail par la suite, le dispositif de capture optique permet avantageusement de lire un code-barres, bidimensionnel ou tridimensionnel, de l'élément vertébral de localisation, afin d'accéder aux informations intégrées à ce code-barres, telles que, entre autres, l'identification de la vertèbre à laquelle l'élément vertébral de localisation est associé de manière spécifique, des données de planification préopératoires relatives à un acte chirurgical que le robot doit réaliser ou assister, etc.

Des caractéristiques additionnelles avantageuses du système conforme à l'invention sont spécifiées aux autres revendications.

Suivant des caractéristiques additionnelles avantageuses du procédé de chirurgie rachidienne précité :
- On planifie en préopératoire au moins un acte chirurgical à réaliser sur la vertèbre et on calcule des données de planification correspondant à cet acte chirurgical, qui sont basées sur les données cartographiques préopératoires ; on équipe l'organe terminal effecteur d'un outil à même de réaliser l'acte chirurgical ; et à partir du calcul du positionnement relatif entre l'organe terminal effecteur et la vertèbre, ainsi qu'à partir des données de planification, on élabore en temps réel des instructions de commande envoyées au robot pour que l'outil de l'organe terminal effecteur soit appliqué directement sur la vertèbre par le bras de manière à réaliser l'acte chirurgical.
- L'acte chirurgical consiste à percer un trou dans la vertèbre ; les données de planification incluent l'orientation de l'axe central du trou par rapport à la vertèbre, le point d'entrée du trou sur la vertèbre, et la profondeur du trou dans la vertèbre ; et l'outil est une mèche de perçage.
- On planifie en préopératoire au moins une action d'assistance à un acte chirurgical à réaliser sur la vertèbre et on calcule des données de planification correspondant à cette action d'assistance, qui sont basées sur les données cartographiques préopératoires ; on équipe l'organe terminal effecteur d'un ancillaire à même d'opérer l'action d'assistance ; et à partir du calcul du positionnement relatif entre l'organe terminal effecteur et la vertèbre, ainsi qu'à partir des données de planification, on élabore en temps réel des instructions de commande envoyées au robot pour que l'ancillaire soit placé vis-à-vis de la vertèbre par le bras de manière à réaliser l'action d'assistance, pendant que l'acte chirurgical est réalisé par le chirurgien, en étant assisté par l'ancillaire.
- Les données cartographiques préopératoires sont issues d'images de scanner des vertèbres.
- La face d'accouplement est fabriquée en utilisant les données cartographiques préopératoires.
- Parmi les données cartographiques préopératoires, on sélectionne en préopératoire des données, dites d'intérêt, qui sont relatives à au moins une région anatomiquement remarquable de la vertèbre, et la face d'accouplement est fabriquée en utilisant les données d'intérêt de manière à, en peropératoire, être appliquée en contact sur mesure avec la matière osseuse de ladite au moins une région anatomiquement remarquable.
- Les données cartographiques préopératoires sont segmentées de manière à former des groupes de données qui sont respectivement relatifs aux vertèbres du patient.
- En peropératoire, le patient est anesthésié et allongé sur le ventre sur une table d'opération qui est fixe par rapport à la station fixe du robot.
- Aucun dispositif d'imagerie par rayon X n'est mis en oeuvre en peropératoire.

L'invention sera mieux comprise à la lecture de la description qui va suivre, donnée uniquement à titre d'exemple et faite en se référant aux dessins sur lesquels :
- la figure 1 est un schéma illustrant une partie d'un système conforme à l'invention ;
- les figures 2 à 4 sont des vues similaires à la figure 1, illustrant respectivement d'autres parties du système ;
- figure 5 est également un schéma illustrant encore d'autres parties du système ;
- la figure 6 est une vue à plus grande échelle d'une des parties montrées à la figure 5 ;
- la figure 7 est une vue en élévation selon la flèche VII de la figure 6 ;
- la figure 8 est une vue similaire à la figure 6, illustrant une variante de la partie correspondante du système ;
- la figure 9 est une vue similaire à la figure 6, illustrant une autre variante de la partie correspondante du système ;
- la figure 10 est une vue en perspective d'un fragment de la partie du système, montrée à la figure 9 ;
- la figure 11 est une vue similaire à la figure 6, illustrant encore une autre variante de la partie correspondante du système ; et
- la figure 12 est une vue similaire à la figure 5, illustrant un mode de réalisation alternatif pour certaines des parties correspondantes du système.

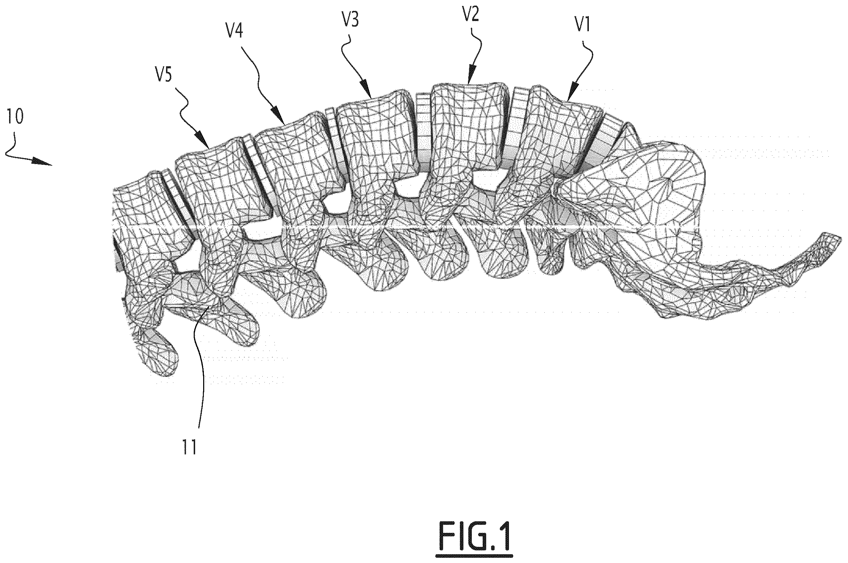

Sur les figures 1 à 7 sont représentées des parties d'un système de chirurgie rachidienne, ce système étant référencé S par la suite. Le système S permet de mettre en œuvre un procédé de chirurgie rachidienne qui va être décrit au fur et à mesure que sont décrites ci-après les parties du système S. A titre d'exemple ré-évoqué par la suite, le procédé vise à implanter des vis pédiculaires dans des vertèbres d'un patient humain. L'exemple illustré aux figures porte sur cinq vertèbres, à savoir des vertèbres qui sont notées V1 à V5 par la suite et qui correspondent aux vertèbres lombaires du patient, mais l'invention n'est évidemment pas limitée par le nombre et/ou la position des vertèbres auxquelles le système S et le procédé de chirurgie rachidienne sont appliqués.

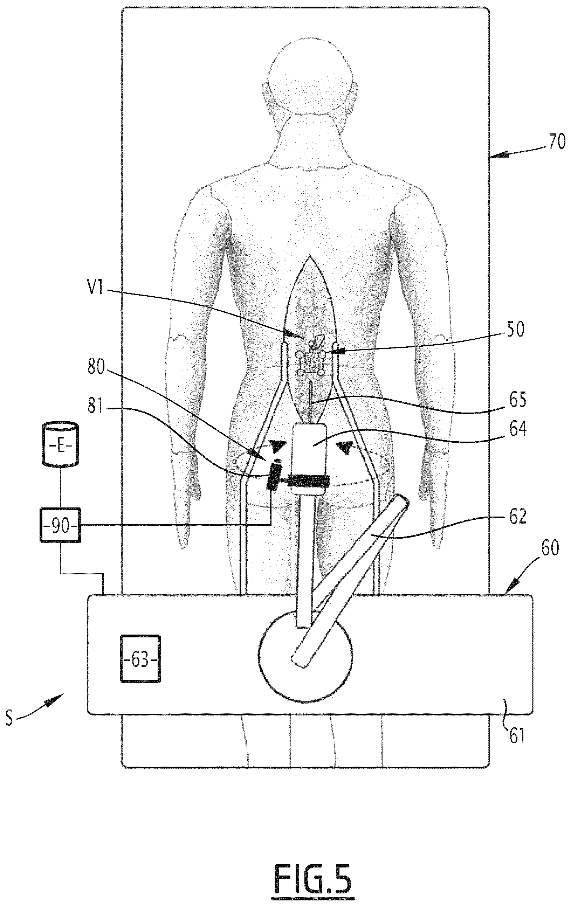

Dans tous les cas, comme expliqué en détail ci-après, le procédé inclut des étapes préopératoires, c'est-à-dire mises en œuvre avant tout acte chirurgical, à proprement parler, appliqué au corps du patient à traiter, et des étapes peropératoires, c'est-à-dire mises en œuvre sur le corps du patient à traiter. Les étapes préopératoires sont mises en œuvre par un opérateur compétent, en utilisant des parties du système S qui vont être décrites notamment en regard des figures 1 à 4. Ces étapes préopératoires peuvent être mises en œuvre plusieurs jours avant les étapes peropératoires qui sont mises en œuvre par un chirurgien ou un professionnel de santé similaire, en utilisant des parties du système S qui vont être décrites en regard des figures 5 à 7. A la différence des étapes préopératoires, les étapes peropératoires sont nécessairement mises en œuvre dans un bloc chirurgical d'un établissement de soins.

Comme illustré par la figure 1, le système S comporte des moyens de cartographie osseuse 10 qui mettent à disposition des données cartographiques préopératoires relatives à une ou plusieurs des vertèbres du patient à opérer, c'est-à-dire aux vertèbres V1 à V5 dans l'exemple considéré. Ces moyens de cartographie 10 permettent de mettre en oeuvre une étape initiale du procédé, dans laquelle l'opérateur dispose des données de cartographie préopératoires précitées, par l'intermédiaire des moyens de cartographie osseuse 10. Dans l'exemple illustré à la figure 1, les données cartographiques préopératoires sont intégrées à une image de scanner 11, appartenant aux moyens de cartographie osseuse 10, c'est-à-dire une image tomographique obtenue par irradiation préopératoire des vertèbres V1 à V5. En pratique, plusieurs images de scanner appartenant aux moyens de cartographie osseuse 10, telles que l'image 11, fournissent les données cartographiques préopératoires précitées. Dans tous les cas, ces données cartographiques préopératoires fournissent un maillage morphologique des structures osseuses des vertèbres V1 à V5, comme illustré schématiquement sur la figure 1. L'acquisition de telles données cartographiques préopératoires relatives à la matière osseuse des vertèbres étant bien connue dans le domaine, elle ne sera pas détaillée ici plus avant.

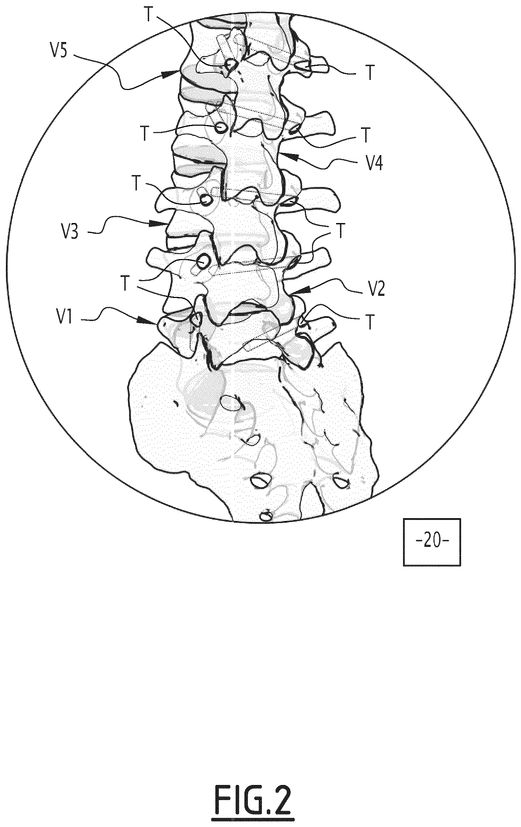

Comme illustré par la figure 2, le système S comporte avantageusement des moyens de planification 20 qui permettent de planifier en préopératoire un ou plusieurs actes chirurgicaux à réaliser sur les vertèbres V1 à V5, ainsi que de calculer des données de planification correspondant à ces actes chirurgicaux, basées sur les données cartographiques préopératoires fournies par les moyens de cartographie osseuse 10. Les moyens de planification 20 sont par exemple un dispositif logiciel à même de traiter les données cartographiques préopératoires. Dans tous les cas, les moyens de planification 20 permettent de mettre en oeuvre une étape du procédé, dite de planification, qui est subséquente à l'étape initiale et dans laquelle l'opérateur planifie les actes chirurgicaux précités. Dans l'exemple illustré à la figure 2, ces actes chirurgicaux consistent à percer des trous T dans les vertèbres V1 à V5, destinés à recevoir les vis pédiculaires mentionnés plus haut. Dans cette étape de planification, l'opérateur détermine, grâce aux moyens de planification 20, les données de planification correspondant aux actes chirurgicaux, en en caractérisant des paramètres de mise en oeuvre. La définition des données de planification repose sur les données cartographiques préopératoires, dans le sens où les données de planification sont définies par rapport aux données cartographiques préopératoires. Dans l'exemple considéré ici en lien avec les trous T à percer dans les vertèbres V1 à V5, l'opérateur détermine ainsi, notamment par calcul, le point d'entrée de chaque trou T sur la vertèbre concernée, l'orientation de l'axe central de chaque trou T par rapport à la vertèbre concernée, et la profondeur de chaque trou T dans la vertèbre concernée, et ce en tenant compte de la taille de la vis pédiculaire à placer dans le trou. Les données de planification sont avantageusement affichées, par les moyens de planification 20, en superposition des données cartographiques préopératoires, comme illustré schématiquement sur la figure 2. En pratique, les spécificités relatives à cette étape de planification et aux moyens de planification 20 ne sont pas limitatives de l'invention, étant remarqué que diverses techniques de planification sont connues dans le domaine.

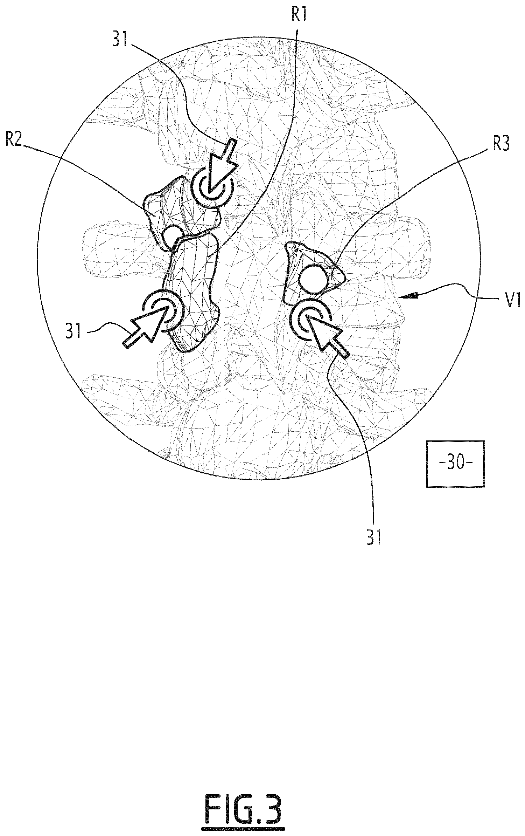

Comme illustré par la figure 3, le système S comporte également de manière avantageuse des moyens de sélection 30 qui permettent de sélectionner en préopératoire, parmi les données cartographiques préopératoires précitées, des données, dites d'intérêt, qui sont relatives à une ou plusieurs régions anatomiquement remarquables de chacune des vertèbres V1 à V5. Les moyens de sélection 30 sont par exemple un dispositif logiciel à même de traiter les données cartographiques préopératoires qui sont fournies par les moyens de cartographie osseuse 10. Dans tous les cas, les moyens de sélection 30 permettent de mettre en œuvre une étape du procédé, qui est nécessairement subséquente à l'étape initiale, tout en étant avantageusement mise en œuvre après l'étape de planification, et dans laquelle les données d'intérêt précitées sont sélectionnées. Sur la figure 3, la vertèbre V1 est ainsi associée à trois régions anatomiquement remarquables, respectivement référencées R1, R2 et R3. Lors de l'étape de sélection, l'opérateur utilise les moyens de sélection 30 pour identifier, parmi les données cartographiques préopératoires, le cas échéant complétées par les données de planification, les régions anatomiquement remarquables de chacune des vertèbres, typiquement au travers d'un affichage de ces données qui permet à l'opérateur de pointer chacune des régions anatomiquement remarquables, comme illustré schématiquement sur la figure 3 où sont dessinés trois pointeurs 31 en forme de flèche. Les moyens de sélection 30 permettent avantageusement d'étendre automatiquement la sélection autour des pointages effectués par l'opérateur, afin d'obtenir ainsi pour chaque vertèbre des portions correspondantes du maillage que forment les données cartographiques préopératoires, de sorte que ces portions du maillage décrivent respectivement les régions anatomiquement remarquables. En variante, la sélection des régions anatomiquement remarquables peut être au moins partiellement opérée automatiquement par les moyens de sélection 30 sous réserve que ces derniers soient programmés en conséquence, en particulier par apprentissage. Dans tous les cas, la partie des données cartographiques préopératoires, qui correspondent à la ou aux régions anatomiquement remarquables de chaque vertèbre, constitue les données d'intérêt précitées et est identifiée de manière ad hoc, en étant notamment conservée en totalité, ces données d'intérêt étant destinés à être exploités lors d'étapes ultérieures du procédé, comme expliqué par la suite. A l'inverse, le reste des données cartographiques préopératoires peut être ignoré ou bien traité pour en limiter la taille informatique. En pratique, la ou les régions anatomiquement remarquables de chaque vertèbre sont typiquement celles avec lesquelles les actes chirurgicaux prévus à l'étape de planification interfèrent, ainsi que, le cas échéant et pour des raisons qui apparaîtront plus loin, le processus épineux de la vertèbre, comme la région R1 pour la vertèbre V1 sur la figure 3, et/ou les lames gauche et droite de la vertèbre, comme les régions R2 et R3 de la vertèbre V1, et/ou les processus articulaires gauche et droit de la vertèbre, et/ou les processus transverses gauche et droit de la vertèbre.

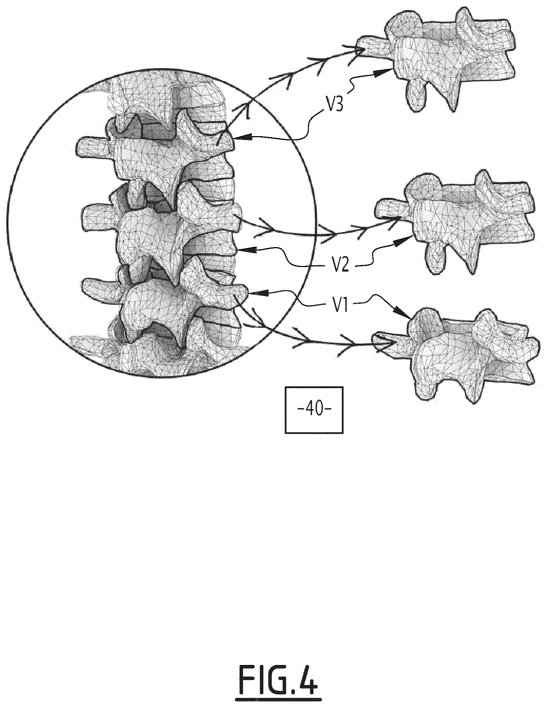

Comme illustré par la figure 4, le système S comporte également de manière avantageuse des moyens de segmentation 40 qui permettent de segmenter les données cartographiques préopératoires, fournies par les moyens de cartographie osseuse 10, de manière que ces données forment des groupes de données qui sont respectivement relatifs aux vertèbres V1 à V5 du patient. Les moyens de segmentation 40 sont par exemple un dispositif logiciel à même de traiter les données cartographiques préopératoires. Dans tous les cas, ces moyens de segmentation 40 permettent de mettre en œuvre une étape du procédé, qui est nécessairement subséquente à l'étape initiale et dans laquelle les données cartographiques préopératoires sont segmentées pour former les groupes de données précités, respectivement relatifs aux vertèbres V1 à V5. Lors de cette étape de segmentation, les moyens de segmentation 40 traitent les données cartographiques préopératoires pour délimiter les contours de chacune des vertèbres V1 à V5 et les distinguer ainsi individuellement vis-à-vis des autres et vis-à-vis du reste de la colonne vertébrale, comme indiqué schématiquement pour les vertèbres V1 à V3 sur la figure 4. Chaque vertèbre ainsi distinguée correspond à une portion correspondante du maillage que forment les données cartographiques préopératoires, cette portion du maillage formant le groupe de données relatif à la vertèbre concernée. On comprend qu'une partie des données cartographiques préopératoires initiales, c'est-à-dire fournies par les moyens de cartographie osseuse 10, ne se retrouvent pas dans les groupes de données précités. Autrement dit, le volume de données, constitué par l'ensemble des groupes de données précités, est moindre que le volume des données cartographiques préopératoires de l'étape initiale. En pratique, le traitement opéré par les moyens de segmentation 40 peut être avantageusement automatique, c'est-à-dire qu'il repose sur des algorithmes ne nécessitant pas l'intervention de l'opérateur, hormis leur éventuelle activation. Quelles que soient les spécificités de mise en œuvre de l'étape de segmentation, cette dernière est opérée avant ou après les étapes de planification et/ou de sélection.

En pratique, un même dispositif logiciel peut avantageusement intégrer les fonctionnalités respectives des moyens de planification 20, des moyens de sélection 30 et des moyens de segmentation 40.

Dans tous les cas, à l'issue des étapes préopératoires que sont l'étape initiale et les étapes de planification, de sélection et de segmentation, les données cartographiques préopératoires, qui, le cas échéant sont avantageusement limitées aux groupes de données issues de l'étape de segmentation et qui intègrent avantageusement la définition des données d'intérêt sélectionnées à l'étape de sélection, ainsi que les données de planification, sont sauvegardées dans un format informatique exportable, bien connu dans le domaine. L'ensemble de toutes ces données est désigné par la référence E par la suite.

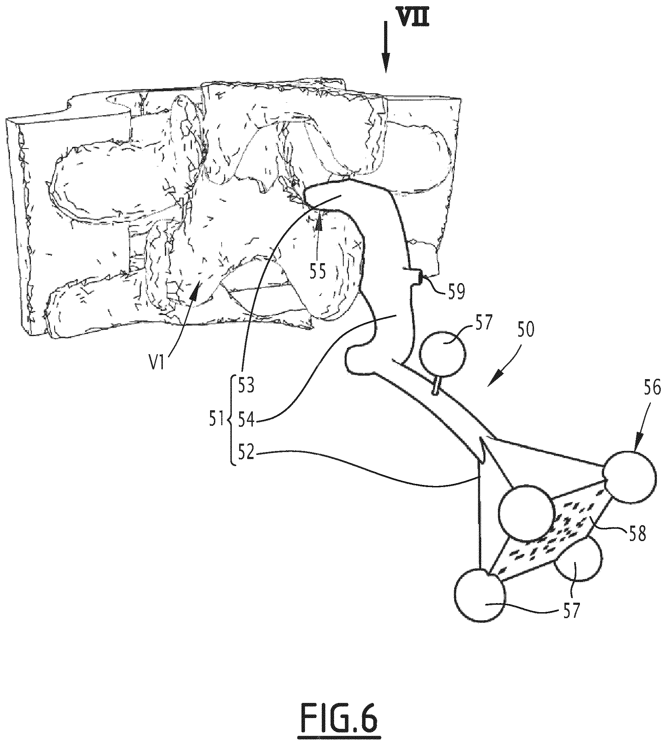

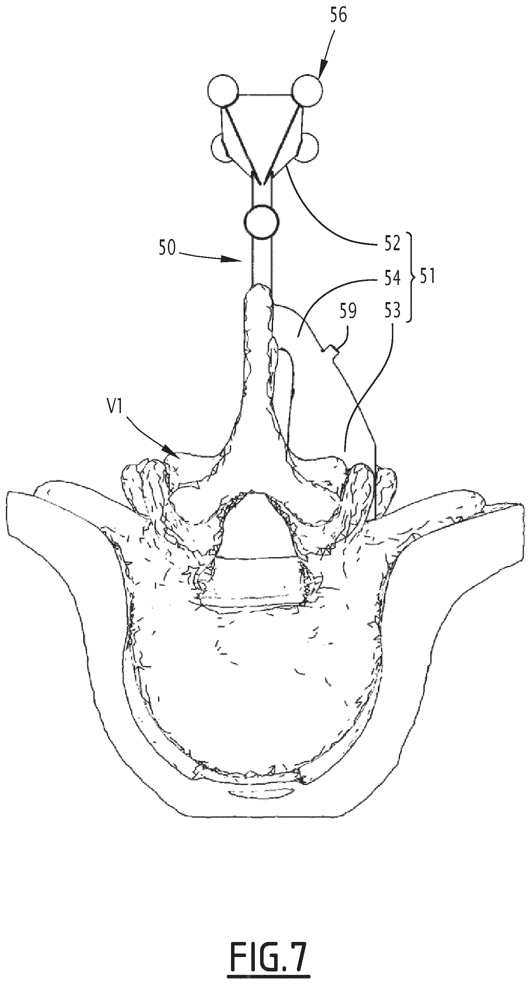

Comme représenté sur la figure 5, le système S comporte par ailleurs un élément vertébral de localisation 50 qui permet de localiser dans l'espace une vertèbre à laquelle cet élément vertébral de localisation est associé. Dans l'exemple considéré sur les figures, l'élément vertébral de localisation 50 est ainsi associé à la vertèbre V1, comme bien visible sur les figures 6 et 7.

L'élément vertébral de localisation 50 est spécifique au patient, plus précisément à une vertèbre de ce patient, ici la vertèbre V1, dans le sens où, comme expliqué par la suite, l'élément vertébral de localisation 50 est conçu de manière personnalisée pour le patient considéré, en étant façonné sur mesure pour correspondre au négatif de la topographie osseuse de certaines zones de la vertèbre V1, qualifiées par la suite de partie osseuse prédéterminée de cette vertèbre V1. Dans la forme de réalisation considérée sur les figures 5 à 7, l'élément vertébral de localisation 50 présente la spécificité d'être hémivertébral, dans le sens où, comme également expliqué par la suite, il est conçu pour être appliqué exclusivement sur une moitié, gauche ou droite, de la vertèbre à laquelle il est associé, sans interagir avec l'autre moitié de cette vertèbre. A titre d'exemple illustré sur les figures 5 à 7, l'élément vertébral de localisation 50 est ainsi spécifiquement adapté à l'hémivertèbre droite de la vertèbre V1.

L'élément vertébral de localisation 50 comporte un corps 51 qui présente des côtés opposés, respectivement proximal et distal. Lorsque l'élément vertébral de localisation 50 est utilisé sur la vertèbre V1, le côté distal de son corps 51 est tourné vers la vertèbre V1, en particulier vers le côté postérieur de cette vertèbre. Dans la forme de réalisation considérée ici, l'élément vertébral de localisation 50 comporte une partie proximale 52 et une partie distale 53, qui sont reliées fixement l'une à l'autre par une partie intermédiaire 54 du corps 51, qui est avantageusement effilée.

Le corps 51 est pourvu, sur son côté distal, d'une face d'accouplement 55 qui est ici délimitée par la partie distale 53. La face d'accouplement 55 permet d'accoupler mécaniquement le corps 51 à la matière osseuse de la vertèbre 1, ici de l'hémivertèbre droite de la vertèbre 1, et est spécifique au patient de manière à pouvoir être appliquée en contact sur mesure avec cette matière osseuse, et ce dans une position fixe unique. A cet effet, la face d'accouplement 55 est morphoadaptée à la vertèbre 1, en étant congruente avec la partie osseuse prédéterminée de la vertèbre V1, cette partie osseuse prédéterminée étant préférentiellement située sur le côté postérieur de la vertèbre. Lorsque la face d'accouplement 55 est appliquée sur l'hémivertèbre droite de la vertèbre V1, la face d'accouplement 55 recouvre ainsi la partie osseuse prédéterminée de la vertèbre V1, en en épousant les reliefs osseux et en coopérant par contact avec ces derniers pour placer l'élément vertébral de localisation 50 sur la vertèbre V1 dans la position fixe unique précitée. L'unicité et la fixité du positionnement résultent de l'ajustement de formes entre la partie osseuse prédéterminée de la vertèbre V1 et la face d'accouplement 55 morphoadaptée.

En pratique, le façonnage de la face d'accouplement 55 est réalisé de manière préopératoire, avantageusement à partir des données cartographiques préopératoires fournies par les moyens de cartographie osseuse 10, qui sont relatives à la vertèbre V1, notamment à la partie osseuse prédéterminée de cette vertèbre. Ces données cartographiques préopératoires sont exploitées de manière préopératoire pour façonner la face d'accouplement 55 de sorte que cette dernière adopte le négatif topographique du maillage morphologique de la partie osseuse prédéterminée de la vertèbre V1. Ce façonnage est mis en oeuvre par toute technique de fabrication appropriée, notamment par fabrication additive, autrement appelée impression tridimensionnelle, et ce de manière connue en soi. Avantageusement, la partie osseuse prédéterminée de la vertèbre V1 correspond à au moins une des régions anatomiquement remarquables de cette vertèbre, qui ont été décrites plus haut en lien avec les moyens de sélection 30 ; cela revient à dire que la face d'accouplement 55 est conçue pour être appliquée en contact sur mesure avec la matière osseuse d'une ou de plusieurs de ces régions anatomiquement remarquables de la vertèbre V1 : les données cartographiques préopératoires qui sont effectivement exploitées pour façonner la face d'accouplement 55 sont ainsi avantageusement incluses dans les données d'intérêt ayant été sélectionnées par les moyens de sélection 30 lors de l'étape de sélection qui a été décrite plus haut.

Le corps 51 de l'élément vertébral 50 est par ailleurs pourvu d'un marqueur 56 qui est ici délimité par la partie proximale 52 du corps 51. Ce marqueur 56 est un marqueur optique tridimensionnel qui, comme expliqué plus en détail par la suite, est conçu pour être observé et suivi par des moyens optiques appropriés. Sur les figures 5 à 7, le marqueur 56 comporte des boules 57 qui sont réparties fixement sur le corps 51. D'autres formes de réalisation, connues en tant que telles dans le domaine, sont envisageables pour le marqueur 56 du moment que ce dernier définit un repère spatial tridimensionnel, qui est lié fixement au corps 51 et par-là à la face d'accouplement 55, de par la structure de ce corps 51. Lorsque l'élément vertébral de localisation 50 est accouplé, par l'intermédiaire de sa face d'accouplement 55, à la vertèbre V1, le repère défini par le marqueur 56 est ainsi lié fixement à la vertèbre V1, et ce avec un positionnement relatif qui est connu du fait de l'accouplement de l'élément vertébral de localisation 50 à la vertèbre V1 dans la position fixe unique précitée.

Suivant une disposition optionnelle avantageuse, qui est illustrée aux figures 5 à 7, le corps 51 de l'élément vertébral de localisation 50 est pourvu d'un code-barres 58 bidimensionnel, qui est ici délimité par la partie proximale 52 du corps 51, avantageusement de manière adjacente au marqueur 56. La forme de réalisation de ce code-barres 58 bidimensionnel n'est pas limitative, le code-barres 58 étant par exemple un code QR ou un code datamatrix. Dans tous les cas, et par définition même, le code-barres 58 intègre des informations qui sont codées sous forme graphique en utilisant des symboles graphiques qui, typiquement, répondent à des normes préétablies. Pour des raisons qui apparaîtront plus loin, le code-barres 58 intègre ainsi des informations relatives à l'identification de la vertèbre à laquelle l'élément vertébral de localisation 50 est accouplé, autrement dit, ici, à l'identification de la vertèbre V1 ; avantageusement, le code-barres 58 intègre également des informations correspondant aux données de planification décrites plus haut, en lien avec les moyens de planification 20, pour la vertèbre V1. Dans tous les cas, l'obtention du code-barres 58 et son intégration au corps 51, en particulier à la partie proximale 52 de ce dernier, relèvent de techniques qui sont connues en soi et qui ne seront donc pas détaillées ici plus avant.

Suivant une autre disposition optionnelle avantageuse, qui est également illustrée sur les figures 5 à 7, le corps 51 de l'élément vertébral de localisation 50 est aussi pourvu d'au moins une canule 59 qui est ici délimitée par la partie intermédiaire 54 du corps 51. La canule 59 permet de recevoir un élément d'ancrage osseux amovible, tel qu'une broche. Ainsi, lors de l'utilisation de l'élément vertébral de localisation 50 et une fois que la face d'accouplement 55 est appliquée sur la vertèbre V1 dans la position fixe unique précitée, le corps 51 peut, si besoin, être verrouillé sur la vertèbre V1 dans cette position fixe unique, en introduisant une broche dans la vertèbre V1 via la canule 59. De cette façon, le corps 51 n'a pas à être tenu manuellement de manière continue lors de l'utilisation de l'élément vertébral de localisation 50, sans courir le risque que ce dernier ne bouge par rapport à la vertèbre. Le corps 51 peut ainsi être avantageusement dépourvu d'élément de préhension correspondant, tel qu'une poignée.

En pratique, le corps 51 est fabriqué de manière préopératoire par toute technique appropriée. Les techniques de fabrication additive, évoquées plus haut pour la fabrication de la face d'accouplement 55, sont envisageables pour fabriquer toute la partie distale 53, voire la totalité du corps 51. En variante, au moins la partie intermédiaire 54 est préfabriquée selon une géométrie fixe, puis sert de support de fixation pour tout ou partie des parties distale 53 et proximale 52 qui sont fabriquées en tenant compte de leurs spécificités en lien avec la vertèbre V1.

Comme représenté sur la figure 5, le système S comporte également un robot 60. Le robot 60 comprend une station fixe 61, qui est fixe par rapport à une table d'opération 70 du système S et qui, dans l'exemple montré sur la figure, forme une table-pont qui enjambe la face supérieure de la table d'opération 70. Le robot 60 comprend également un bras 62 qui est déplaçable de manière motorisée par rapport à la station fixe 61, en étant commandé en déplacement par des organes moteurs qui sont intégrés au robot 60 et pilotés par une unité de commande 63 du robot. La forme de réalisation du bras robotisé 62, en particulier pour ce qui concerne ses capacités cinématiques et ses organes moteurs, n'est pas limitative du moment que le bras 62 est capable de participer à la chirurgie rachidienne. Le robot 60 comporte en outre un organe terminal effecteur 64 qui est porté par le bras 62, typiquement à l'extrémité de ce dernier qui est opposée à la station fixe 61. Comme détaillé par la suite, l'organe terminal effecteur 64 est adapté pour être appliqué en peropératoire sur les vertèbres V1 à V5. Dans la forme de réalisation illustrée aux figures, l'organe terminal effecteur 64 est équipé d'un outil 65 qui est à même de réaliser au moins un des actes chirurgicaux qui ont été planifiés lors de l'étape de planification, décrite plus haut. Dans le cadre de l'exemple chirurgical en lien avec les trous T à percer dans les vertèbres V1 à V5, l'outil 65 est typiquement une mèche capable de percer le ou les trous T dans la vertèbre V1. En pratique, le robot 60 est un appareil qui, en tant que tel, est disponible dans le commerce.

Egalement comme représenté sur la figure 5, le système S comporte aussi un dispositif de capture optique 80 qui est au moins partiellement porté par l'organe terminal effecteur 64. Dans le mode de réalisation considéré sur la figure 5, le dispositif de capture optique 80 comporte une caméra tridimensionnelle 81 qui est portée par l'organe terminal effecteur 64. Dans le présent document, on entend par « caméra tridimensionnelle » un capteur optique qui capture des données tridimensionnelles grâce à une technique optique telle que le profilage laser, la projection de franges ou le temps de vol. Parmi les techniques optiques par temps de vol, on peut notamment mentionner la télédétection par laser, usuellement appelée LIDAR (acronyme de l'expression anglaise « light detection and ranging »). Ainsi, suivant une forme de réalisation préférentielle, la caméra tridimensionnelle 81 est un capteur optique utilisant la triangulation laser pour calculer la distance le séparant d'un objet à partir de la déformation d'une ligne laser projetée sur cet objet ; la caméra tridimensionnelle 81 peut ainsi avantageusement utiliser la technologie LIDAR pour déterminer le positionnement dans l'espace d'un dispositif dont elle reconnait la forme. Plus généralement, la caméra tridimensionnelle 81 du système S présente l'avantage d'être au plus près de l'élément vertébral de localisation 50, ce qui lui confère une bonne précision dans le positionnement de cet élément vertébral de localisation 50. Par ailleurs cette proximité permet également de réduire l'encombrement de l'élément vertébral de localisation 50 dont les boules 57 n'ont pas besoin d'être aussi espacées que si elles étaient observées à plusieurs mètres du site de l'intervention et donc de l'élément vertébral de localisation 50.

Quelle que soit la forme de réalisation de la caméra tridimensionnelle 81, elle permet, lorsque l'élément vertébral de localisation 50 est accouplé à la vertèbre V1, d'observer le marqueur 56 de cet élément vertébral de localisation 50 et en déduire en temps réel des données de positionnement concernant le positionnement relatif entre ce marqueur 56 et l'organe terminal effecteur 64. Bien entendu, cela repose sur le fait que la position de la caméra tridimensionnelle 81 sur l'organe terminal effecteur 64 est connue de manière préétablie et renseignée de manière ad hoc dans la caméra tridimensionnelle 81, cette position pouvant être soit fixe dès lors que la caméra tridimensionnelle 81 est solidarisée fixement à l'organe terminal effecteur 64, soit commandable en déplacement selon une cinématique précise et contrôlée, comme illustré par les flèches en pointillés sur la figure 5.

Dans la forme de réalisation de la figure 5, le dispositif de capture optique 80 est avantageusement adapté pour, lorsque l'élément vertébral de localisation 50 est accouplé à la vertèbre V1, observer et lire le code-barres 58 de l'élément vertébral de localisation 50. A cet effet, le dispositif de capture optique 80 comporte un capteur optique, par exemple bidimensionnel, à même de lire les symboles graphiques du code-barres 58 et d'en déduire les informations intégrées à ce code-barres. De multiples formes de réalisation, connues en tant que telles dans la technique, sont envisageables pour ce capteur optique, étant entendu que ce dernier est avantageusement intégré à la caméra tridimensionnelle 81.

Egalement comme représenté sur la figure 5, le système S comporte des moyens de traitement 90, typiquement électroniques, qui sont conçus pour traiter à la fois les données de l'ensemble E et les données de positionnement déterminées par la caméra tridimensionnelle 81 ou, plus généralement, par le dispositif de capture optique 80, ainsi que pour avantageusement traiter les informations du code-barres 58, lues par le dispositif de capture optique 80. Les moyens de traitement 70 sont avantageusement conçus pour également élaborer des instructions de commande à destination du robot 60, envoyées à l'unité de commande 63 de ce dernier. La transmission de données entre les moyens de traitement 90, le dispositif de capture optique 80 et l'unité de commande 63 du robot 60 est opérée par tout moyen approprié, notamment filaire ou non-filaire. En pratique, les moyens de traitement 90 comprennent notamment un microprocesseur et sont typiquement intégrés à une unité informatique, ces aspects matériels des moyens de traitement 90 n'étant pas limitatifs. Les capacités fonctionnelles des moyens de traitement 90 apparaîtront plus clairement au travers de la suite de la description, qui est relative aux étapes peropératoires du procédé mis en oeuvre en utilisant le système S.

Lors d'une première étape peropératoire du procédé, le patient à opérer est anesthésié après avoir été allongé sur le ventre sous la table d'opération 70, comme illustré sur la figure 5. Le chirurgien réalise alors l'abord de la colonne vertébrale du patient de manière à mettre à nu, sans altérer, une zone de la vertèbre V1. La zone mise à nu est ainsi dégagée de toute partie molle, telle que la peau, la chair, etc., pour laisser la matière osseuse de cette zone de la vertèbre V1 à l'air libre. La zone ainsi mise à nu inclut a minima la partie osseuse prédéterminée de la vertèbre V1, sur laquelle l'élément vertébral de localisation 50 est conçu pour être placé dans la position fixe unique précitée, moyennant la coopération par contact entre cette partie osseuse prédéterminée de la vertèbre V1 et la face d'accouplement 55 de l'élément vertébral de localisation 50. La zone mise à nu peut avantageusement être limitée à la seule partie osseuse prédéterminée de la vertèbre V1, en étant ainsi exposée par une voie d'abord que l'on peut qualifier de mini invasive et qui, ici, ne concerne qu'un seul côté latéral, à savoir le côté droit, de la vertèbre V1, moyennant une incision latéralisée réduite de la colonne vertébrale du patient. En variante, la zone mise à nu peut, bien entendu, être davantage étendue, en étant alors exposée par une voie d'abord ouverte large, le cas échéant en concernant les deux côtés latéraux de la colonne vertébrale.

Lors d'une deuxième étape peropératoire, le chirurgien utilise l'élément vertébral de localisation 50, en appliquant la face d'accouplement 55 de ce dernier sur la zone mise à nu de la vertèbre V1 de manière à placer le corps 51 sur la vertèbre 1 dans la position fixe unique ayant été définie plus haut. L'élément vertébral de localisation 50 est alors dans la configuration illustrée sur les figures 5 à 7. Une fois accouplé à la vertèbre 1, ici à l'hémivertèbre droite de cette vertèbre 1, l'élément vertébral de localisation 50 peut être lâché par le chirurgien, tout en restant fixement en place sur la vertèbre 1, de manière stable. Le chirurgien peut alors facilement contrôler visuellement le site opératoire, d'autant que le corps 51 est particulièrement compact, notamment grâce à l'effilement de sa partie intermédiaire 54.

Lors d'une troisième étape peropératoire, après que le robot 60, le dispositif de capture optique 80 et les moyens de traitement 90 aient été activés, la caméra tridimensionnelle 81 est placée en regard du marqueur 56 de l'élément vertébral de localisation 50. Par observation du marqueur 56 de l'élément vertébral de localisation 50 accouplé à la vertèbre V1, la caméra tridimensionnelle 81 détermine en temps réel le positionnement relatif entre ce marqueur 56 et l'organe terminal effecteur 64 du robot 60, comme expliqué plus haut. Les données de positionnement correspondantes, ainsi déterminées par la caméra tridimensionnelle 81, sont transmises instantanément aux moyens de traitement 90 qui, à partir de ces données de positionnement et à partir des données cartographiques préopératoires de l'ensemble E, calculent en temps réel le positionnement relatif entre l'organe terminal effecteur 64 et la vertèbre V1. Pour les moyens de traitement 90, cela revient à, dans le repère spatial qui est défini par le marqueur 56 et qui est lié fixement à la vertèbre V1 selon un positionnement connu, calculer en temps réel la position de l'organe terminal effecteur 64 et comparer cette position avec la région de l'espace occupée par la vertèbre V1 telle que modélisée par les données cartographiques préopératoires relatives à cette vertèbre V1. Cela suppose évidemment que la vertèbre à laquelle est accouplé l'élément vertébral de localisation 50 soit identifiée par les moyens de traitement 90 comme étant la vertèbre V1, ce qui résulte avantageusement de la lecture du code-barres 58 par le dispositif de capture optique 80 qui transmet les informations de ce code-barres 58 aux moyens de traitement 90 ; en alternative, cela peut également résulter d'une saisie ad hoc de l'information correspondante dans les moyens de traitement 90 par le chirurgien. Dans tous les cas, on comprend que la notion de « temps réel » implique que le dispositif de capture optique 80 et les moyens de traitement 90 sont intrinsèquement capables de contrôler et piloter le bras 62 du robot 60 à une vitesse adaptée aux éventuels mouvements de la vertèbre V1 et donc du marqueur 56 observée par la caméra tridimensionnelle 81, ces mouvements éventuels étant liés par exemple à la respiration du patient, à des spasmes du patient ou à un choc externe inopiné subit par la table d'opération 70. Cette capacité de traitement en temps réel est liée à une fréquence importante d'actualisation de la détermination des positionnements relatifs concernés. A titre d'exemple, cette fréquence est de l'ordre de 100 kHz. Le système S permet ainsi de corriger en temps réel le déplacement de l'organe terminal effecteur 64 lors de son application sur la vertèbre V1 en fonction des mouvements éventuels de cette dernière.

Dans le prolongement des considérations qui précèdent, les moyens de traitement 90 sont avantageusement prévus pour, lors de la troisième étape peropératoire, exploiter également les données de planification ayant été définies plus haut, de façon à élaborer en temps réel, à partir de ces données de planification et à partir du calcul opéré par les moyens de traitement 90 quant au positionnement relatif entre l'organe terminal effecteur 64 et la vertèbre V1, des instructions de commande qui sont envoyées à l'unité de commande 63 du robot 60 pour faire appliquer l'outil 65 de l'organe terminal effecteur 64 sur la vertèbre V1 par le bras 62 de manière à réaliser au moins l'un des actes chirurgicaux correspondants aux données de planification. Dans le cadre de l'exemple chirurgical en lien avec les trous T à percer dans les vertèbres V1 à V5, la mèche formant l'outil 65 est ainsi appliquée par le bras 62 directement sur la vertèbre V1 pour y percer un trou dont l'orientation de l'axe central, le point d'entrée et la profondeur respectent ceux planifiés lors de l'étape préopératoire de planification. Lors de l'application de cette mèche sur la vertèbre V1, tout mouvement inopiné de cette dernière induit, par l'intermédiaire de la caméra tridimensionnelle 81 et des moyens de traitement 90, un recalcul positionnel permettant d'actualiser en conséquence les instructions de commande qu'envoient les moyens de traitement 90 à l'unité de commande 63 du robot 60. En pratique, les données de planification sont fournies aux moyens de traitement 90 soit par extraction directement depuis l'ensemble de donnée E, soit préférentiellement par l'intermédiaire du dispositif de capture optique 80 lisant le code-barres 58 dans lequel ont été intégrées des informations correspondant aux données de planification relatives à la vertèbre V1.

Pendant tout l'actionnement du robot 50 au cours de la troisième étape peropératoire, le marqueur 56 reste facilement et efficacement observable par la caméra tridimensionnelle 81 du fait que cette dernière est montée sur l'organe terminal effecteur 64. Cette proximité opérationnelle entre le marqueur 56 et la caméra tridimensionnelle 81 est particulièrement bénéfique sur la précision de suivi optique, tout en limitant significativement le risque d'interruption de suivi optique par occlusion entre le marqueur 56 et la caméra tridimensionnelle 81. Par ailleurs, la complexité des calculs opérés par les moyens 90 reste modérée car elle peut avantageusement se limiter au calcul de positionnement de trois points respectivement associés aux boules 57, typiquement les centres respectifs des boules 57, permettant le repérage dans l'espace de la vertèbre V1 et du positionnement relatif de l'organe terminal effecteur 64 par rapport à ladite vertèbre V1.

Le procédé se poursuit ensuite en tenant compte de la planification préopératoire, en répétant si besoin la troisième étape peropératoire afin de percer dans la vertèbre V1 un ou plusieurs autres des trous T, puis en répétant les deuxième et troisième étapes peropératoires mais en les appliquant à successivement chacune des vertèbres V2 à V5 pour y percer les différents autres trous T. Bien entendu, pour répéter la deuxième étape peropératoire en l'appliquant aux vertèbres V2 à V5, il est nécessaire de disposer, pour chacune de ces vertèbres V2 à V5, d'un élément vertébral de localisation qui est fonctionnellement similaire à l'organe vertébral de localisation 50 décrit jusqu'ici, mais qui est propre à la vertèbre concernée, en ayant sa face d'accouplement qui est spécifique à la vertèbre concernée et en ayant son code-barres dont les informations concernent la vertèbre concernée.

A l'issue du procédé, les vertèbres V1 à V5 se retrouvent percées avec les trous T qui ont été planifiés lors de l'étape préopératoire de planification, chacun de ces trous ayant été percé par le robot 60.

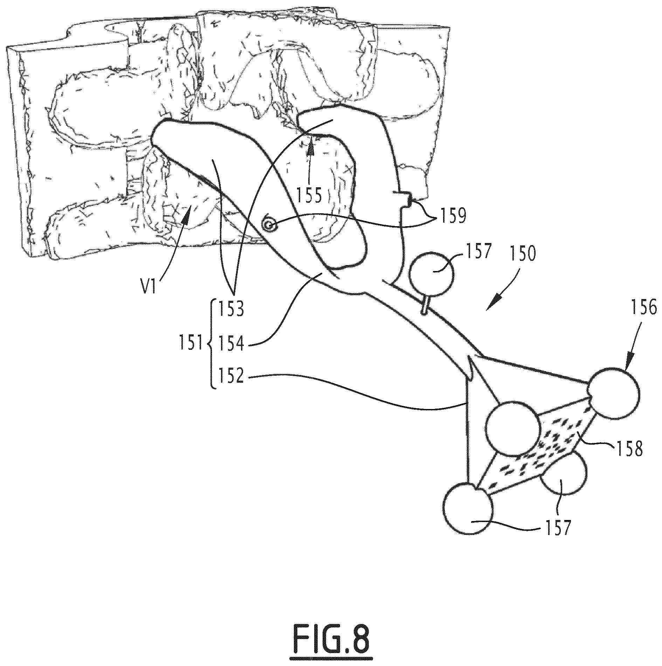

Sur la figure 8 est représentée une variante de l'élément vertébral de localisation 50, référencée 150. L'élément vertébral de localisation 150 est fonctionnellement similaire à l'organe vertébral de localisation 50, mais s'en distingue structurellement par le fait que sa face d'accouplement 155, qui est fonctionnellement similaire à la face d'accouplement 55, est conçue pour être appliquée en contact sur mesure avec simultanément la matière osseuse de la moitié gauche de la vertèbre V1 et la matière osseuse de la moitié droite de cette vertèbre V1. A cet effet, l'élément vertébral de localisation 150 comporte un corps 151 dont une partie distale 153, qui délimite la face d'accouplement 155, est répartie de part et d'autre du processus épineux de la vertèbre V1 lorsque l'élément vertébral de localisation 150 est accouplé par sa face d'accouplement 155 à la vertèbre V1, en particulier aux lames gauche et droite de cette dernière, comme illustré à la figure 8. Bien entendu, les gestes chirurgicaux qui sont mis en oeuvre aux première et deuxième étapes peropératoires sont adaptés en conséquence.

Dans la forme de réalisation envisagée à la figure 8, le corps 151 de l'élément vertébral de localisation 150 comporte une partie proximale 152, qui est fonctionnellement et structurellement similaire à la partie proximale 52, en étant notamment pourvue d'un marqueur 156 et d'un code-barres 158 qui sont respectivement similaires au marqueur 56 et au code-barres 58. Le corps 151 comporte également une partie intermédiaire 154 qui relie l'une à l'autre les parties proximale 152 et distale 153 en formant une arche, qui chevauche le processus épineux de la vertèbre V1 lorsque l'élément vertébral de localisation 250 est accouplé à la vertèbre V1 et qui intègre deux canules 159 individuellement similaires à la canule 59.

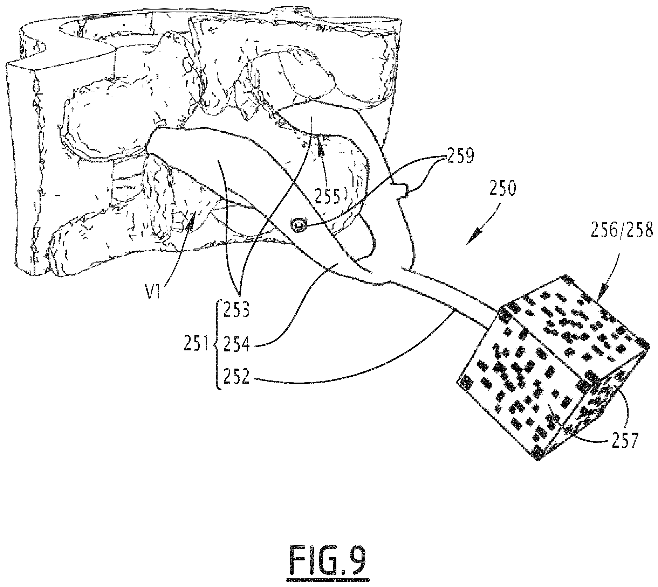

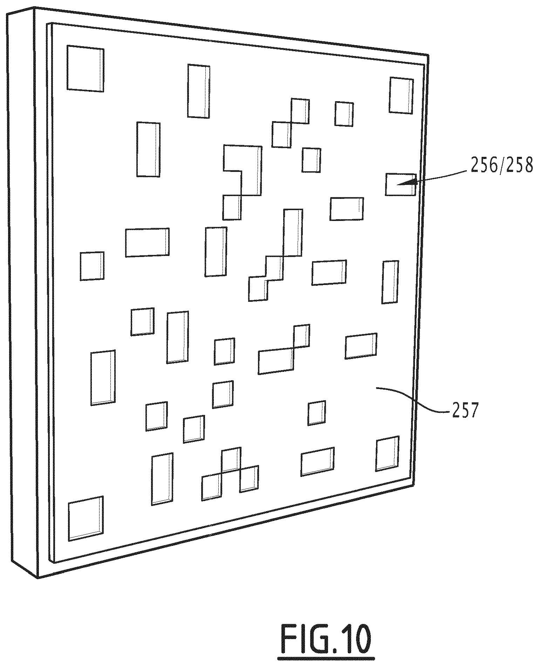

Sur la figure 9 est représentée une variante de l'élément vertébral de localisation 150, référencée 250. L'élément vertébral de localisation 250 est fonctionnellement similaire à l'élément vertébral 50 ou 150, mais se distingue structurellement de l'élément vertébral 150 par, d'une part, la forme de réalisation de son marqueur optique tridimensionnel, qui est référencé 256 et qui est fonctionnellement similaire au marqueur 56 ou 156, et, d'autre part, le fait que son code-barres 258, qui est fonctionnellement similaire au code-barres 58 ou 158, n'est pas bidimensionnel, mais tridimensionnel, en constituant en partie le marqueur 256. A cet effet, des faces 257 du marqueur 256, observables par la caméra tridimensionnelle 81 du dispositif de capture optique 80 lorsque l'élément vertébral de localisation 250 est accouplé à la vertèbre V1, intègrent chacune des motifs tridimensionnels formant conjointement le code-barres 258, l'une de ses faces 257 étant représentée seule à la figure 10. De par leur relief tridimensionnel, les motifs tridimensionnels des faces 257 assurent une fonction similaire aux boules 57 du marqueur 56, notamment un repère spatial qui est lié fixement au marqueur 256 et qui est localisable par la caméra tridimensionnelle 81. De par leur graphisme, les motifs tridimensionnels des faces 257 permettent également d'assurer une fonction similaire au code-barres 58 ou 158, notamment en intégrant des informations identiques à celles décrites plus haut pour le code-barres 58.

Dans la forme de réalisation considérée sur la figure 9, le marqueur 256 et le code-barres 258 sont délimités par une partie proximale 252 d'un corps 251 de l'élément vertébral 250, cette partie proximale 252 étant fonctionnellement similaire à la partie proximale 52 ou 152 des éléments vertébraux de localisation 50 et 150. Ce corps 250 comporte une partie distale 253, qui est fonctionnellement et structurellement similaire à la partie distale 153, en étant notamment pourvue d'une face d'accouplement 255 similaire à la face d'accouplement 155, ainsi qu'une partie intermédiaire 254 qui est fonctionnellement et structurellement similaire à la partie intermédiaire 154, en étant notamment pourvue de canules 259 qui sont similaires aux canules 159.

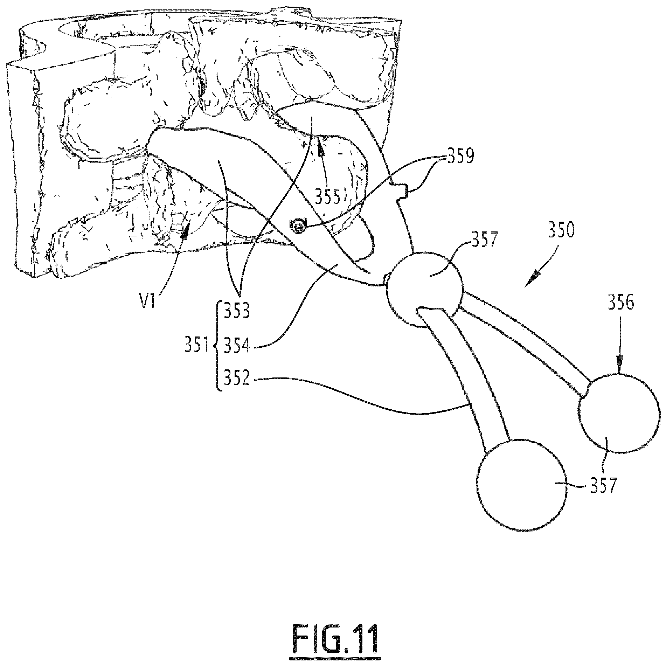

Sur la figure 11 est représentée une variante de l'élément vertébral de localisation 150, référencée 350. L'élément vertébral de localisation 350 est fonctionnellement similaire à l'élément vertébral de localisation 50, 150 ou 250, à la différence près que l'élément vertébral de localisation 350 est dépourvu de code-barres, tel que les codes-barres 58, 158 et 258. L'élément vertébral de localisation 350 est ainsi utilisable en remplacement de l'élément vertébral de localisation 50, 150 ou 250 dès lors qu'on renonce à intégrer, sous forme de code-barres, des informations similaires à celles détaillées plus haut en lien avec le code-barres 58. Comme évoqué précédemment, ces informations sont alors à fournir aux moyens de traitement 90 autrement qu'en lisant un code-barres intégré à l'élément vertébral de localisation 350, notamment en renseignant ces informations directement dans les moyens de traitement 90, le cas échéant par extraction depuis l'ensemble de données E pour une partie de ces informations.

Dans la forme de réalisation considérée sur la figure 11, l'élément vertébral de localisation 350 comporte un marqueur optique tridimensionnel, qui est référencé 356 et qui, tout en étant fonctionnellement similaire au marqueur 56 ou 156, est structurellement différent de ces derniers, en comportant des boules 357 qui sont fonctionnellement similaires aux boules 57 mais qui sont ici agencées différemment les unes par rapport aux autres. Le marqueur 356 est ici délimité par une partie proximale 352 d'un corps 351 de l'élément vertébral de localisation 350. Ce corps 351 comporte une partie distale 353, qui est fonctionnellement et structurellement similaire à la partie distale 153, en étant notamment pourvue d'une face d'accouplement 355 qui est similaire à la face d'accouplement 155, ainsi que d'une partie intermédiaire 354, qui est fonctionnellement et structurellement similaire à la partie 154, en étant notamment pourvue de canules 359 similaires aux canules 159.

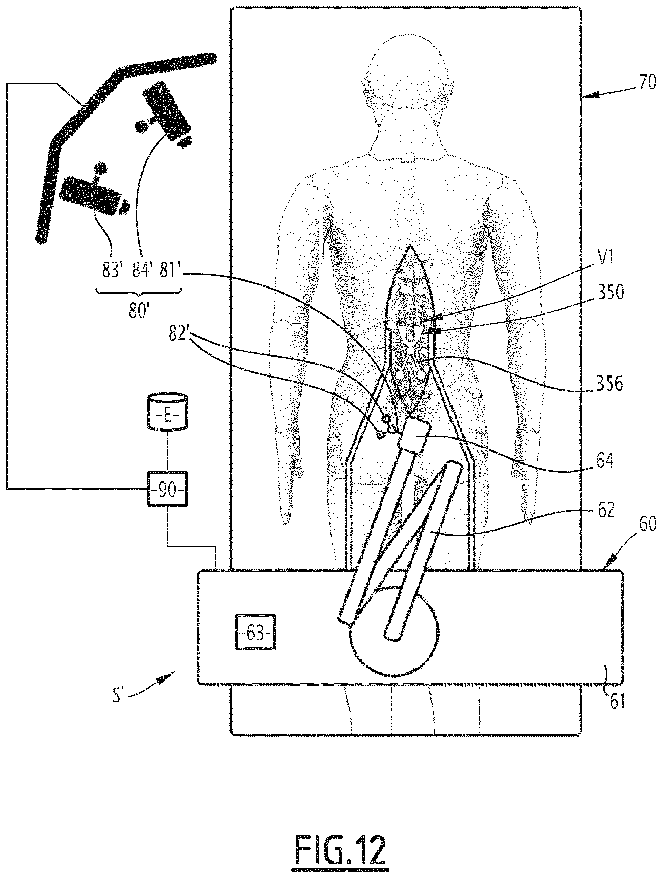

Sur la figure 12 est représenté un mode de réalisation alternatif du système S, référencé S'. Ce système S' comporte l'élément vertébral de localisation 350, le robot 60, la table d'opération 70 et les moyens de traitement 90 du système S, mais, à la différence de ce dernier, est dépourvu du dispositif de capture optique 80 au profit d'un dispositif de capture optique 80'. Le dispositif de capture optique 80' est fonctionnellement similaire au dispositif de capture optique 80, mais s'en distingue structurellement. A cet effet, le dispositif de capture optique 80' comporte un marqueur optique tridimensionnel, qui est référencé 81' et qui est porté, en particulier fixement, par l'organe terminal effecteur du robot 60, comme bien visible sur la figure 12. Bien que distinct du marqueur 356 de l'élément vertébral de localisation 350, le marqueur 81' a une finalité similaire au marqueur 356 mais appliquée à l'organe terminal effecteur 64, dans le sens où le marqueur 81' permet de repérer dans l'espace l'organe terminal effecteur 64 de manière optique. Pour ce faire, le marqueur 81' comporte par exemple des boules 82', similaires aux boules 357 du marqueur 356, mais d'autres formes de réalisation sont envisageables pour le marqueur 81'. Dans tous les cas, le système de capture optique 80' comporte également au moins deux caméras bidimensionnelles 83' et 84' qui, comme illustré schématiquement sur la figure 12, permettent, lorsque l'élément vertébral de localisation 350 est accouplé à la vertèbre V1, d'observer selon des angles d'observation respectifs, qui sont différents l'un de l'autre, à la fois le marqueur 356 et le marqueur 81'. Les caméras bidimensionnelles 83' et 84' sont conçues pour déduire, à partir de leur observation des marqueurs 356 et 81', le positionnement relatif entre le marqueur 356 et l'organe terminal effecteur 64. On comprend que les données de positionnement ainsi déterminées par les deux caméras bidimensionnelles 83' et 84' sont similaires à celles déterminées par la seule caméra tridimensionnelle 81. Les données de positionnement déterminées par les caméras bidimensionnelles 83' et 84' sont fournies aux moyens de traitement 90 pour permettre à ces derniers de calculer en temps réel le positionnement relatif entre l'organe terminal effecteur 64 et la vertèbre V1, comme expliqué plus haut pour le système S.

En variantes non représentées, l'acte chirurgical réalisé par le robot 60 du système S ou S' sur les vertèbres V1 à V5 ne se limite pas au perçage de trous. Plus généralement, le ou les actes chirurgicaux réalisables par le robot 60 consistent à appliquer une énergie entamant la matière osseuse des vertèbres, cette énergie pouvant être de nature mécanique, radiative, laser, etc. L'outil 65 de l'organe terminal effecteur 64 du robot 60 est choisi en conséquence, en étant approprié à la nature de l'acte chirurgical à réaliser par le robot 60. Au cours d'une même intervention chirurgicale, cet outil 65 est d'ailleurs interchangeable.

Également à titre de variante non représentée, plutôt que d'utiliser le robot 60 pour réaliser un acte chirurgical comme envisagé ci-dessus, le robot 60 peut, aussi bien dans le système S que dans le système S', être utilisé pour assister un acte chirurgical réalisé par le chirurgien, par exemple pour guider un geste du chirurgien appliquant sur les vertèbres un outil de perçage ou autre. Dans ce cas, lors de l'étape préopératoire de planification, on calcule des données de planification qui correspondent à une action d'assistance à l'acte chirurgical et qui sont basées sur les données cartographiques préopératoires. Puis, lors de l'étape peropératoire au cours de laquelle l'acte chirurgical est réalisé par le chirurgien, l'outil 65 de l'organe terminal effecteur 64 est remplacé par un ancillaire à même de réaliser l'action d'assistance correspondante. L'action d'assistance est opérée par le robot 60 : à partir des données de planification correspondant à l'action d'assistance, ainsi qu'à partir du calcul du positionnement relatif entre l'organe terminal effecteur 64 et la vertèbre V1, des instructions de commande sont élaborées en temps réel par les moyens de traitement 90 et envoyées au robot 60 pour que l'ancillaire soit placé vis-à-vis de la vertèbre V1 par le bras 62 de manière à réaliser l'action d'assistance, pendant que l'acte chirurgical est réalisé par le chirurgien, en étant assisté par l'ancillaire.

Enfin, divers aménagements et options aux systèmes S et S', ainsi qu'au procédé décrits jusqu'ici sont envisageables. En particulier, les différentes formes de réalisation mentionnées plus haut pour des parties respectives du système S ou S' peuvent être associées selon toutes les combinaisons possibles pour donner de nouveaux modes de réalisation. Par exemple, les parties distale et intermédiaire du corps 251 ou 351 des éléments vertébraux de localisation 250 et 350 sont remplaçables par les parties distale 53 et intermédiaire 54 du corps 51. De même, l'élément vertébral de localisation 50 peut être associé au code barre tridimensionnel schématisé en figure 10 en lieu et place du code barre bidimensionnel 58. Dans cette configuration, le dispositif de capture optique 80 ne comporte plus de capteur optique et se limite à une caméra tridimensionnelle permettant d'une part de déterminer la position de l'élément vertébral 50 par rapport à l'organe terminal effecteur 64 et d'autre part de lire les informations contenues dans le code barre tridimensionnel précité.

## Revendications

1. Système de chirurgie rachidienne (S ; S'), comportant :
- des moyens de cartographie osseuse (10) adaptés pour mettre à disposition des données cartographiques préopératoires relatives à une ou des vertèbres (V1 à V5) d'un patient,
- un élément vertébral de localisation (50 ; 150 ; 250 ; 350) qui est pourvu :
- d'une face d'accouplement (55 ; 155 ; 255 ; 355) qui est spécifique au patient, en étant congruente avec une partie osseuse prédéterminée de la ou d'une des vertèbres (V1), de manière à pouvoir accoupler l'élément vertébral de localisation à la vertèbre (V1) dans une position fixe unique, en appliquant la face d'accouplement en contact sur mesure avec la matière osseuse de cette vertèbre de manière que la face d'accouplement recouvre la partie osseuse prédéterminée de la vertèbre, en épousant des reliefs osseux de la partie osseuse prédéterminée et en coopérant par contact avec ces reliefs osseux pour placer l'élément vertébral de localisation sur la vertèbre dans ladite position fixe unique, et
- d'un marqueur (56 ; 156 ; 256 ; 356) optique tridimensionnel, définissant un repère spatial tridimensionnel qui est lié fixement à la face d'accouplement (55 ; 155 ; 255 ; 355) de sorte que, lorsque l'élément vertébral de localisation (50 ; 150 ; 250 ; 350) est accouplé à la vertèbre (V1), ledit repère spatial tridimensionnel est lié fixement à la vertèbre selon un positionnement relatif résultant de l'accouplement de l'élément vertébral de localisation à la vertèbre dans ladite position fixe unique,
- un robot (60) comprenant un bras (62) qui est mobile par rapport à une station fixe (61) du robot, ainsi qu'un organe terminal effecteur (64) qui est porté par le bras et qui est adapté pour être appliqué en peropératoire sur la vertèbre (V1),
- un dispositif de capture optique (80 ; 80'), qui est au moins partiellement porté par l'organe terminal effecteur (64) et qui est adapté pour, lorsque l'élément vertébral de localisation (50 ; 150 ; 250 ; 350) est accouplé à la vertèbre (V1), observer le marqueur (56 ; 156 ; 256 ; 356) de l'élément vertébral de localisation et en déduire en temps réel des données de positionnement concernant le positionnement relatif entre ce marqueur et l'organe terminal effecteur, et
- des moyens de traitement (90) adaptés pour, à partir desdites données cartographiques préopératoires et desdites données de positionnement, déterminer en temps réel le positionnement relatif entre l'organe terminal effecteur (64) et la vertèbre (V1), en calculant, dans ledit repère spatial tridimensionnel, la position de l'organe terminal effecteur et en comparant cette position de l'organe terminal effecteur avec une région de l'espace occupée par la vertèbre telle que modélisée par lesdites données cartographiques préopératoires.

2. Système de chirurgie rachidienne suivant la revendication 1,
dans lequel le système de chirurgie rachidienne (S ; S') comporte également des moyens de planification (20), qui sont adaptés pour planifier en préopératoire au moins un acte chirurgical à réaliser sur la vertèbre (V1) et pour calculer des données de planification correspondant à cet acte chirurgical, basées sur les données cartographiques préopératoires,
dans lequel l'organe terminal effecteur (64) est équipé d'un outil (65) à même de réaliser l'acte chirurgical,
et dans lequel les moyens de traitement (90) sont adaptés pour élaborer en temps réel, à partir du calcul du positionnement relatif entre l'organe terminal effecteur (64) et la vertèbre (V1), calculé par les moyens de traitement, et à partir desdites données de planification, des instructions de commande envoyées au robot (60) pour faire appliquer l'outil (65) de l'organe terminal effecteur directement sur la vertèbre par le bras (62) de manière à réaliser ledit acte chirurgical.

3. Système de chirurgie rachidienne suivant l'une des revendications 1 ou 2,
dans lequel l'élément vertébral de localisation (50 ; 150 ; 250) est pourvu d'un code-barres (58 ; 158 ; 258) intégrant des informations qui sont relatives à l'identification de la vertèbre (V1) à laquelle l'élément vertébral de localisation est à accoupler,
et dans lequel le dispositif de capture optique (80) est adapté pour, lorsque l'élément vertébral de localisation (50 ; 150 ; 250) est accouplé à la vertèbre (V1), observer et lire le code-barres (58 ; 158 ; 258).

4. Système de chirurgie rachidienne suivant les revendications 2 et 3 prises ensemble, dans lequel le code-barres (58 ; 158 ; 258) intègre également des informations correspondant aux données de planification.

5. Système de chirurgie rachidienne suivant l'une des revendications 3 ou 4, dans lequel le code-barres (58 ; 158) est bidimensionnel, en étant par exemple un code QR ou un code datamatrix.

6. Système de chirurgie rachidienne suivant l'une des revendications 3 ou 4, dans lequel le code-barres (258) est tridimensionnel et constitue au moins en partie le marqueur (256) de l'élément vertébral de localisation (250).

7. Système de chirurgie rachidienne suivant l'une quelconque des revendications précédentes, dans lequel le dispositif de capture optique (80) comporte une caméra tridimensionnelle (81), qui est portée par l'organe terminal effecteur (64) et qui est adaptée pour déterminer lesdites données de positionnement.

8. Système de chirurgie rachidienne suivant l'une quelconque des revendications 1 à 6, dans lequel le dispositif de capture optique (80') comporte :
- un marqueur (81') optique tridimensionnel, qui est distinct du marqueur (56 ; 156 ; 256 ; 356) de l'élément vertébral de localisation (50 ; 150 ; 250 ; 350) et qui est porté par l'organe terminal effecteur (64), et
- au moins deux caméras bidimensionnelles (83', 84') qui sont adaptées pour, lorsque l'élément vertébral de localisation est accouplé à la vertèbre (V1), observer selon des angles d'observation respectifs, qui sont différents l'un de l'autre, à la fois le marqueur de l'élément vertébral de localisation et le marqueur du dispositif de capture optique et en déduire en temps réel lesdites données de positionnement.

9. Système de chirurgie rachidienne suivant l'une quelconque des revendications précédentes, dans lequel la face d'accouplement (55) est conçue pour être appliquée en contact sur mesure avec la matière osseuse d'uniquement une moitié, gauche ou droite, de la vertèbre (V1).

10. Système de chirurgie rachidienne suivant l'une quelconque des revendications 1 à 8, dans lequel la face d'accouplement (155 ; 255 ; 355) est conçue pour être appliquée en contact sur mesure avec simultanément la matière osseuse de la moitié gauche de la vertèbre (V1) et la matière osseuse de la moitié droite de la vertèbre.

## Patentansprüche

1. Wirbelsäulenchirurgiesystem (S; S'), aufweisend:
- Mittel zur Knochenkartierung (10), die dazu geeignet sind, präoperative kartographische Daten bereitzustellen, die sich auf einen oder mehrere Wirbel (V1 bis V5) eines Patienten beziehen,
- ein Wirbel-Lokalisierungselement (50; 150; 250; 350), das versehen ist mit:
- einer Kopplungsfläche (55; 155; 255; 355), die patientenspezifisch ist, indem sie mit einem vorbestimmten Knochenteil des oder von einem der Wirbel (V1) kongruent ist, so dass das Wirbel-Lokalisierungselement am Wirbel (V1) in einer einzigen festen Position gekoppelt werden kann, indem die Kopplungsfläche in maßgeschneidertem Kontakt an das Knochenmaterial dieses Wirbels angelegt wird, so dass die Kopplungsfläche den vorbestimmten Knochenteil des Wirbels bedeckt, indem sie sich an Knochenreliefs des vorbestimmten Knochenteils anschmiegt und durch Kontakt mit diesen Knochenreliefs zusammenwirkt, um das Wirbel-Lokalisierungselement in der einzigen festen Position auf dem Wirbel zu platzieren, und
- einem dreidimensionalen optischen Marker (56; 156; 256; 356), der ein dreidimensionales räumliches Bezugssystem definiert, das fest mit der Kopplungsfläche (55; 155; 255; 355) verbunden ist, so dass, wenn das Wirbel-Lokalisierungselement (50; 150; 250; 350) am Wirbel (V1) gekoppelt ist, das dreidimensionale räumliche Bezugssystem fest mit dem Wirbel gemäß einer relativen Position verbunden ist, die aus der Kopplung des Wirbel-Lokalisierungselements am Wirbel in dieser einzigen festen Position resultiert,
- einen Roboter (60), der einen Arm (62) aufweist, der relativ zu einer festen Station (61) des Roboters beweglich ist, sowie ein ausführendes Endorgan (64), das von dem Arm getragen wird und dazu geeignet ist, intraoperativ am Wirbel (V1) angelegt zu werden,
- eine optische Erfassungsvorrichtung (80; 80'), die zumindest teilweise von dem ausführenden Endorgan (64) getragen wird und die dazu geeignet ist, wenn das Wirbel-Lokalisierungselement (50; 150; 250; 350) am Wirbel (V1) gekoppelt ist, den Marker (56; 156; 256; 356) des Wirbel-Lokalisierungselements zu beobachten und daraus in Echtzeit Positionsdaten abzuleiten, die die relative Position zwischen diesem Marker und dem ausführenden Endorgan betreffen, und
- Verarbeitungsmittel (90), die dazu geeignet sind, anhand der präoperativen kartografischen Daten und der Positionsdaten in Echtzeit die relative Position zwischen dem ausführenden Endorgan (64) und dem Wirbel (V1) durch Berechnen der Position des ausführenden Endorgans in dem dreidimensionalen räumlichen Bezugssystem und durch Vergleichen dieser Position des ausführenden Endorgans mit einem Bereich des Raums, der von dem Wirbel eingenommen wird, wie von den präoperativen kartografischen Daten modelliert, zu bestimmen.

2. Wirbelsäulenchirurgiesystem nach Anspruch 1,
wobei das Wirbelsäulenchirurgiesystem (S; S') ebenfalls Planungsmittel (20) aufweist, die geeignet sind, präoperativ mindestens einen an dem Wirbel (V1) durchzuführende operativen Eingriff zu planen und diesem operativen Eingriff entsprechende Planungsdaten zu berechnen, die auf den präoperativen kartographischen Daten basieren,
wobei das ausführende Endorgan (64) mit einem Werkzeug (65) ausgestattet ist, das imstande ist, den chirurgischen Eingriff durchzuführen,
und wobei die Verarbeitungsmittel (90) geeignet sind, in Echtzeit aus der Berechnung der relativen Position zwischen dem ausführenden Endorgan (64) und dem Wirbel (V1), berechnet von den Verarbeitungsmitteln, und aus den Planungsdaten Steueranweisungen auszuarbeiten, die an den Roboter (60) gesendet werden, damit das Werkzeug (65) des ausführenden Endorgans vom Arm (62) direkt an den Wirbel angelegt wird, um den chirurgischen Eingriff durchzuführen.

3. Wirbelsäulenchirurgiesystem nach einem der Ansprüche 1 oder 2,
wobei das Wirbel-Lokalisierungselement (50; 150; 250) mit einem Strichcode (58; 158; 258) mit Informationen versehen ist, die sich auf die Identifizierung des Wirbels (V1) beziehen, an dem das lokalisierende Wirbelelement gekoppelt werden soll,
und wobei die optische Erfassungsvorrichtung (80) geeignet ist, wenn das Wirbel-Lokalisierungselement (50; 150; 250) am Wirbel (V1) gekoppelt ist, den Strichcode (58; 158; 258) zu beobachten und zu lesen.

4. Wirbelsäulenchirurgiesystem nach Anspruch 2 und 3 zusammengenommen, wobei der Strichcode (58; 158; 258) ebenfalls Informationen enthält, die den Planungsdaten entsprechen.

5. Wirbelsäulenchirurgiesystem nach einem der Ansprüche 3 oder 4, wobei der Strichcode (58; 158) zweidimensional ist, indem er beispielsweise ein QR-Code oder ein Datamatrix-Code ist.

6. Wirbelsäulenchirurgiesystem nach einem der Ansprüche 3 oder 4, wobei der Strichcode (258) dreidimensional ist und zumindest teilweise den Marker (256) des Wirbel-Lokaliserungselements (250) bildet.

7. Wirbelsäulenchirurgiesystem nach einem der vorhergehenden Ansprüche, wobei die optische Erfassungsvorrichtung (80) eine dreidimensionale Kamera (81) aufweist, die vom ausführenden Endorgan (64) getragen wird und die dazu geeignet ist, die Positionsdaten zu bestimmen.

8. Wirbelsäulenchirurgiesystem nach einem der Ansprüche 1 bis 6, wobei die optische Erfassungsvorrichtung (80') aufweist:
- einen dreidimensionalen optischen Marker (81'), der sich von dem Marker (56; 156; 256; 356) des Wirbel-Lokaliserungselements (50; 150; 250; 350) unterscheidet und der von dem ausführenden Endorgan (64) getragen wird, und
- mindestens zwei zweidimensionale Kameras (83', 84'), die dazu geeignet sind, um, wenn das Wirbel-Lokalisierungselement am Wirbel (V1) gekoppelt ist, aus jeweiligen Beobachtungswinkeln, die sich voneinander unterscheiden, sowohl den Marker des Wirbel-Lokalisierungselements als auch den Marker der optischen Erfassungsvorrichtung zu beobachten und daraus in Echtzeit die Positionsdaten abzuleiten.

9. Wirbelsäulenchirurgiesystem nach einem der vorhergehenden Ansprüche, wobei die Kopplungsfläche (55) so gestaltet ist, um in maßgeschneidertem Kontakt mit dem Knochenmaterial von nur einer Hälfte, links oder rechts, des Wirbels (V1) angelegt zu sein.

10. Wirbelsäulenchirurgiesystem nach einem der Ansprüche 1 bis 8, wobei die Kopplungsfläche (155; 255; 355) so gestaltet ist, um gleichzeitig in maßgeschneidertem Kontakt mit dem Knochenmaterial der linken Hälfte des Wirbels (V1) und der rechten Hälfte des Wirbels angelegt zu sein.

## Claims

1. A spine surgery system (S; S'), including:
- bone mapping means (10) which provides preoperative mapping data relative to one or more vertebrae (V1 to V5) of a patient,
- a vertebral localization element (50; 150; 250; 350) which is provided with:
- a mating surface (55; 155; 255; 355) which is specific to the patient, being congruent with a predetermined bony part of the vertebra or one of the vertebrae (V1), so as to be able to couple the vertebral localization element to the vertebra (V1) in a unique fixed position, applying the mating surface in customized contact with the bony material of this vertebra so that the mating surface covers the predetermined bony part of the vertebra, by matching the bony reliefs of the predetermined bone portion and by cooperating by contact with these bony reliefs to place the vertebral localization element on the vertebra in said unique fixed position, and
- a three-dimensional optical marker (56; 156; 256; 356), defining a three-dimensional spatial marker which is fixedly linked to the mating surface (55; 155; 255; 355) so that, when the vertebral element (50; 150; 250; 350) is coupled to the vertebra (V1), said three-dimensional spatial marker is fixedly linked to the vertebra according to a relative position resulting from the coupling of the vertebral localization element to the vertebra in said unique fixed position,
- a robot (60) comprising an arm (62) which is movable relative to a fixed station (61) of the robot, as well as an end effector member (64) which is carried by the arm and which is able to be applied intraoperatively on the vertebra (V1),
- an optical sensor device (80; 80'), which is at least partially carried by the end effector member (64) and which is able, when the vertebral localization element (50; 150; 250; 350) is coupled to the vertebra (V1), to observe the marker (56; 156; 256; 356) of the vertebral localization element and deducing therefrom, in real time, the positioning data concerning the relative position between this marker and the end effector member, and
- processing means (90) which, from said preoperative mapping data and said positioning data, calculates, in real time, the relative position between the end effector member (64) and the vertebra (V1), by calculating, in said three-dimensional spatial reference, the position of the end effector member and by comparing this position of the end effector member with a region of space occupied by the vertebra as modeled by said preoperative mapping data.

2. The spine surgery system according to claim 1,
wherein the spine surgery system (S; S') also includes a planning means (20), which is able to plan preoperatively at least one surgical act to be performed on the vertebra (V1) and to calculate planning data corresponding to this surgical act, based on the preoperative mapping data,
wherein the end effector member (64) is equipped with a tool (65) able to perform the surgical act,
and wherein the processing means (90) elaborates in real time, from the calculation of the relative position between the end effector member (64) and the vertebra (V1), calculated by the processing means, and from said planning data, control instructions sent to the robot (60) to cause the tool (65) of the end effector member to be applied directly to the vertebra by the arm (62) so as to perform said surgical act.

3. The spine surgery system according to one of claims 1 or 2,
wherein the vertebral localization element (50; 150; 250) is provided with a barcode (58; 158; 258) integrating the information relative to the identification of the vertebra (V1) to which the vertebral localization element is to be coupled,
and wherein the optical sensor device (80) is able to, when the vertebral localization element (50; 150; 250) is coupled to the vertebra (V1), observe and read the barcode (58; 158; 258).

4. The spine surgery system according to claims 2 and 3 taken together, wherein the barcode (58; 158; 258) also incorporates the information corresponding to the planning data.

5. The spine surgery system according to one of claims 3 or 4, wherein the barcode (58; 158) is two-dimensional, being for example a QR code or a Data Matrix code.

6. The spine surgery system according to one of claims 3 or 4, wherein the barcode (258) is three-dimensional and constitutes at least in part the marker (256) of the vertebral localization element (250).

7. The spine surgery system according to any one of the preceding claims, wherein the optical sensor device (80) includes a three-dimensional camera (81), which is carried by the end effector member (64) and which determines said positioning data.

8. The spine surgery system according to any one of claims 1 to 6, wherein the optical sensor device (80 ') includes:
- a three-dimensional optical marker (81'), which is distinct from the marker (56; 156; 256; 356) of the vertebral localization element (50; 150; 250; 350) and which is carried by the end effector member (64), and
- at least two, two-dimensional cameras (83', 84') which are able, when the vertebral localization element is coupled to the vertebra (V1), to observe, according to respective viewing angles, which are different the one from the other, at the same time the marker of the vertebral localization element and the marker of the optical sensor device and deduce said positioning data in real time.

9. The spine surgery system according to any one of the preceding claims, wherein the mating surface (55) is designed to be applied in customized contact with the bony material of only one half, left or right, of the vertebra (V1).

10. The spine surgery system according to any one of claims 1 to 8, wherein the mating surface (155; 255; 355) is designed to be applied in customized contact with the bony material of the left half of the vertebra (V1) and the bony material of the right half of the vertebra.
